# EUROPÄISCHE PATENTSCHRIFT

(11) **EP 0 562 450 B1**
(45) Veröffentlichungstag und Bekanntmachung des Hinweises auf die Patenterteilung: **09.12.1998**
(21) Anmeldenummer: 93104422.6
(22) Anmeldetag: 18.03.1993
(51) Int. Cl.: C07C 31/125, C07C 69/80, C07C 29/17, C07C 45/50, C07C 47/21, C07C 45/74

(54) **Decylalkoholgemische, daraus erhältliche Phthalsäureester und ihre Verwendung als Weichmacher**
Mixtures of decyl alcohols, phthalic acid esters obtained from these and their application as plasticisers
Mélanges d'alcools décycliques, esters de l'acide phtalique obtenus de ceux-ci et leur utilisation comme agents plastifiants

(30) Priorität: 27.03.1992 DE 4210028
(43) Veröffentlichungstag der Anmeldung: 29.09.1993
(73) Patentinhaber: Celanese GmbH, 60439 Frankfurt (DE)
(72) Erfinder: Bahrmann, Helmut, Dipl.-Chem., W-4236 Hamminkeln 3 (DE); Greb, Wolfgang, Dipl.-Ing., W-4220 Dinslaken (DE); Heymanns, Peter, Dipl.-Chem., W-4300 Essen 1 (DE); Lappe, Peter, Dipl.-Chem., W-4220 Dinslaken (DE); Müller, Thomas, Dipl.-Ing., W-4220 Dinslaken (DE); Szameitat, Jürgen, Dr. Dipl.-Chem., W-4230 Wesel (DE); Wiebus, Ernst, W-4200 Oberhausen (DE)

(56) Entgegenhaltungen:
- EP-A- 0 052 999
- EP-A- 0 094 456
- EP-A- 0 366 089
- DE-A- 2 627 354

## Beschreibung

Die Erfindung betrifft Gemische isomerer Decylalkohole, ein Verfahren zu ihrer Herstellung, die aus diesen Alkoholen erhaltenen Phthalsäureester und deren Verwendung als Weichmacher.

Ester der Phthalsäure finden in großem Umfang als Weichmacher, insbesondere für Polyvinylchlorid, Verwendung. Als Alkoholkomponente werden vorwiegend primäre Alkohole mit 8 bis 10 Kohlenstoffatomen eingesetzt, größte Bedeutung unter ihnen hat gegenwärtig das 2-Ethylhexanol. Phthalsäureester kurzkettigerer Alkohole führen zu Weichmachern mit guter Gelierkraft. Nachteilig ist, verglichen mit längerkettigen Verbindungen, jedoch ihre höhere Flüchtigkeit. Längerkettige Ester gelieren dagegen langsamer und haben eine schlechtere Kältebeständigkeit.

Die Eigenschaften der Phthalsäureester-Weichmacher werden außer durch die Größe des Alkoholmoleküls auch durch die Verzweigung der Kohlenstoffkette beeinflußt. So ergeben wenig verzweigte Alkohole Esterweichmacher, die wegen ihrer hohen Kälteflexibilität besonders geschätzt sind. Weitgehend lineare Alkohole mit 8 bis 10 Kohlenstoffatomen im Molekül gewinnen daher als Alkoholkomponente zunehmend an Bedeutung. Voraussetzung für ihren Einsatz ist, daß sie in großen Mengen und kostengünstig zur Verfügung stehen.

Nach der deutschen Patentschrift 2 855 421 werden als Weichmacher Phthalate von C₉-Alkoholen eingesetzt, die durch Oxoreaktion von C₈-Olefinen, Hydrierung des Reaktionsproduktes und Veresterung der C₉-Alkohole mit Phthalsäureanhydrid erhalten werden. 3 bis 20 Gew.-% der Ausgangsolefine sollen in jeder Molekülkette ein Isobutanskelett, weniger als 3 Gew.-% der Olefine quaternären Kohlenstoff aufweisen und mehr als 90 Gew.-% der Gesamtmenge der Olefine als n-Octene, Monomethylheptene und Dimethylhexene vorliegen. Ferner soll das Gewichtsverhältnis der Gesamtmenge der n-Octene und Monomethylheptene zu den Dimethylhexenen mehr als 0,8 betragen.

Phthalsäureester auf Basis von C₁₀-Alkoholen sind Gegenstand der europäischen Patentanmeldung 0 366 089. Die C₁₀-Alkohole werden in Form eines Gemisches eingesetzt, das man durch Hydroformylierung einer Butenfraktion, Aldolkondensation des erhaltenen Aldehydgemisches und anschließende Hydrierung gewinnt.

Auch die europäische Patentanmeldung EP-A-0 094 456 offenbart die Herstellung von C₁₀-Alkohol-Gemischen durch Hydroformylierung von Butenen in homogener Phase, Aldolkondensation des erhaltenen C₅-Aldehydgemisches und anschließende Hydrierung.

Ein anderer Weg zur Gewinnung von Di-Decylphthalatgemischen ist in der europäischen Patentanmeldung 04 24 767 beschrieben. Die Herstellung der Ester erfolgt nach einem mehrstufigen Verfahren durch Dimerisierung von Butengemischen, Hydroformylierung und Hydrierung des resultierenden Octengemisches zu einem Nonanolgemisch, Dehydratisierung des Nonanolgemisches unter Bildung eines Nonengemisches und Hydroformylierung und Hydrierung des Nonengemisches unter Bildung eines Decanolgemisches.

Die bekannten Verfahren erfüllen in wirtschaftlicher und technischer Hinsicht noch nicht alle Anforderungen, die an einen im großen Maßstab ausgeübten Prozeß gestellt werden, sei es, daß die Ausgangsstoffe nicht in ausreichender Menge und/oder nicht kostengünstig zur Verfügung stehen oder daß die Umwandlung der Ausgangsstoffe in die Alkohole an zu aufwendige Prozesse gebunden ist.

Es bestand daher die Aufgabe, ein Verfahren zu entwickeln, das von Rohstoffen ausgeht, die preiswert zur Verfügung stehen und die in technisch einfacher Weise in die gewünschten Alkohole überführt werden können.

Die Erfindung besteht in einem Verfahren zur Herstellung von Gemischen isomerer Decylalkohole erhalten durch Hydroformylierung von Butadien zu einem Aldehydgemisch, Abtrennung des erhaltenen Aldehydgemischs aus dem Reaktionsprodukt, Kondensation des Aldehydgemisches unter Bildung eines Aldolgemisches und Abtrennung und Hydrierung des Aldolgemisches zu einem Gemisch isomerer Decylalkohole. Dieses Verfahren ist dadurch gekennzeichnet, daß die Hydroformylierung des Butadiens in Gegenwart von Wasser und wasserlöslichen Rhodium-Komplexverbindungen als Katalysator erfolgt, die sulfonierte Aryl-, Alkyl- oder Alkylaryldiphosphine als Liganden enthalten.

Das Ausgangsgemisch zur Herstellung des Gemisches isomerer Decylalkohole, Butadien, ist nicht nur durch Dehydrierung von Butan oder Buten leicht zugänglich, sondern fällt auch bei der Herstellung von Ethylen durch thermische Spaltung von Leichtbenzin oder höheren Kohlenwasserstoffen zwangsweise in erheblichen Mengen an. Man isoliert es aus den C₄-Crackschnitten des Pyrolyseproduktes, z.B. durch Flüssig-Flüssig-Extraktion mit einem selektiven Lösungsmittel wie Acetonitril, Dimethylformamid oder N-Methylpyrolidon.

Zur Hydroformylierung setzt man Butadien in der heute handelsüblichen Form ein, d.h. in einer Reinheit von mindestens 99,5 Gew%.

Im Rahmen der vorliegenden Erfindung wird die Hydroformylierung des Butadiens in einem heterogenen Zweiphasensystem durchgeführt. Sie ist prinzipiell aus der DE-PS-26 27 354 bekannt. Ein solcher Prozeß ist charakterisiert durch das Vorliegen einer organischen Phase, die das Ausgangsolefin und das Reaktionsprodukt enthält und eine wäßrige Phase, in der der Katalysator gelöst ist. Als Katalysatoren finden wasserlösliche Rhodiumkomplexverbindungen Anwendung, die als Liganden sulfonierte Alkyl-, Aryl- oder Alkylaryldiphosphine enthalten. Ihre Herstellung ist bekannt, vgl. z.B. DE-PS 26 27 354 und DD-PS 259 194. Die Reaktion erfolgt bei Temperaturen von 60 bis 150°C, vorzugsweise 90 bis 120 °C und unter Drücken im Bereich von 0,4 bis 30, insbesondere 1 bis 10 MPa. Die Rhodiumkonzentration beträgt 20 bis 2000 Gew.-ppm, vorzugsweise 50 bis 500 Gew.-ppm, bezogen auf die wäßrige Katalysatorlösung; je mol Rhodium setzt man 4 bis 100 mol wasserlösliches Phosphin ein. Das Volumverhältnis von wäßriger zu organischer Phase beträgt 0,1 bis 10:1.

Der Butadienumsatz wird deutlich erhöht, wenn man der wäßrigen Katalysatorlösung ein Phasentransferreagenz (Lösungsvermittler) zusetzt. Bewährt haben sich insbesondere kationische Lösungsvermittler der allgemeinen Formel [A-N(R¹R²R³)]⁺E⁻, in der A für einen geradkettigen oder verzweigten Alkylrest mit 6 bis 25 Kohlenstoffatomen steht, R¹,R²,R³ gleich oder verschieden sind und geradketrige oder verzweigte Alkylreste mit 1 bis 4 Kohlenstoffatomen bedeuten und E insbesondere für Sulfat, Tetrafluorborat, Acetat, Methosulfat, Benzolsulfonat Alkylbenzolsulfonat, Toluolsulfonat, Lactat oder Citrat, steht.

Die Hydroformylierung des Butadiens mit Rhodium als Katalysator liefert im zweiphasigen Reaktionssystem in hoher Ausbeute eine Gemisch überwiegend gesättigter Aldehyde. Das Verhältnis von gesättigem zu ungesättigtem Aldehyd läßt sich in weiten Grenzen durch Variation des Wasserstoffanteile im Synthesegas beeinflussen. Zunehmender Wasserstoffpartialdruck begünstigt die Rildung gesättigter Aldehyde.

Das Aldehydgemisch enthält 90 und mehr Prozent n-Valeraldehyd und nur in untergeordnetem Maße andere Aldehyde, darunter i-Valeraldehyd, Dialdehyde und ferner Nebenprodukte. Mit den im erfindungsgemäßen Verfahren eingesetzten wasserlöslichen Rhodiumkatalysatoren, die als Liganden wasserlösliche, sulfonierte Alkyl-, Aryl- und Alkylaryldiphosphine erzielt man besonders hohe n/i-Verhältnisse.

Nach Beendigung der Hydroformylierung wird das Aldheydgemisch vom Katalysator, von den nichtumgesetzten Reaktionsteinehmern und den übrigen Reaktionsprodukten abgetrennt. Bei Umsetzung in homogener Phase ist die Destillation das übliche Trennverfahren. Aufgrund der Durchführung der Hydroformylierung in einem Zweiphasensystem können Produkt und Katalysator durch einfache Phasentrennung voneinander geschieden werden. Dieses Verfahren ist in der technischen Durchführung wesentlich einfacher und wegen der fehlenden thermischen Belastung auch erheblich schonender als die destillative Isolierung des Aldehydgemischs. Die Hydroformylierung des Butadiens kann diskontinuierlich oder kontinuierlich erfolgen. Eine hohe Selektivität zu n-Valeraldehyd erzielt man, wenn lediglich ein Teil des eingesetzten Butadiens in Aldehyd überführt und der Rest rezirkuliert wird.

Die Aldolkondensation der als Gemisch vorliegenden Aldehyde erfolgt auf konventionellem Wege unter der Einwirkung basischer Katalysatoren. Eine Vorbehandlung der Aldehyde, z.B. eine spezielle Reinigung, ist nicht erforderlich. Als Katalysatoren finden Alkalicarbonate oder Alkalihydroxide, insbesondere Verbindungen des Natriums oder Kaliums und Amine, vorzugsweise tertiäre Amine, wie Triethylamin, Tri-n-propylamin, Tri-n-butylamin, Anwendung. Man arbeitet bei Temperaturen von 60 bis 160°C, insbesondere 80 bis 130°C und bei Normaldruck oder bei bis etwa 1 MPa erhöhtem Druck. Die Reaktionszeit beträgt wenige Minuten bis zu mehreren Stunden und ist insbesondere abhängig von Katalysatortyp und Reaktionstemperatur. Auf Grund seiner höheren Reaktionsgeschwindigkeit dimerisiert vornehmlich n-Valeraldehyd mit sich selbst oder mit isomeren Valeraldehyden zu Decenalen, eine Kondensation der verzweigten C₅-Aldehyde untereinander tritt dagegen völlig in den Hintergrund.

Das durch Kondensation erhaltene Aldehydgemisch wird anschließend zu einem Decylalkoholgemisch hydriert. Die Wasserstoffanlagerung erfolgt in bekannter Weise in Gegenwart von Katalysatoren. Geeignet sind z.B. Hydrierkatalysatoren auf Basis von Nickel, Chrom oder Kupfer. Üblicherweise liegt die Hydriertemperatur zwischen 100 und 180°C und der Druck zwischen 1 und 10 MPa. Zur Reinigung wird das Decylalkoholgemisch destilliert. Es eignet sich vorzüglich als Alkoholkomponente in Phthalsäureestern, die als Weichmacher verwendet werden sollen. Die Herstellung der Phthalsäureester ist bekannt [vgl. Ullmann, Encyclopädie der Technischen Chemie (1979), Bd. 18, Seite 536 ff]. Zweckmäßig setzt man Phthalsäureanhydrid mit dem Decylalkoholgemisch im Molverhältnis 1 : 2 in einer Stufe um. Die Reaktionsgeschwindigkeit kann durch Katalysatoren und/oder durch Erhöhung der Reaktionstemperatur erhöht werden. Um das Gleichgewicht in Richtung der Esterbildung zu verschieben, ist es erforderlich, das gebildete Wasser aus dem Reaktionsgemisch zu entfernen.

Die aus dem erfindungsgemäßen Decylalkoholgemisch erhaltenen Phthalate zeichnen sich als Weichmacher durch hervorragende Kälteeigenschaften aus.

## Patentansprüche

1. Verfahren zur Herstellung von Gemischen isomerer Decylalkohole, erhalten durch Hydroformylierung von Butadien zu einem Aldehydgemisch, Abtrennung des erhaltenen Aldehydgemischs aus dem Reaktionsprodukt, Kondensation des Aldehydgemisches unter Bildung eines Aldolgemischs und Abtrennung und Hydrierung des Aldolgemischs zu einem Gemisch isomerer Decylalkohole, dadurch gekennzeichnet, daß die Hydroformylierung des Butadiens in Gegenwart von Wasser und wasserlöslichen Rhodium-Komplexverbindungen als Katalysator erfolgt, die sulfonierte Aryl-, Alkyl- oder Alkylaryldiphosphine als Liganden enthalten.

2. Verfahren zur Herstellung von Gemischen isomerer Decylalkohole nach Anspruch 1, dadurch gekennzeichnet, daß die Hydroformylierung des Butadiens in Gegenwart von Wasser und wasserlöslichen Rhodium-Diphosphin-Komplexverbindungen als Katalysator bei Temperaturen von 60 bis 150°C und unter Drücken von 0,4 bis 30 MPa erfolgt, das Volumverhältnis von wäßriger zu organischer Phase 0,1 bis 10:1 und die Rhodiumkonzentration in der wäßrigen Katalysatorlösung 20 bis 2000 ppm beträgt und je mol Rhodium 4 bis 100 mol Phosphin eingesetzt werden.

3. Verfahren zur Herstellung von Gemischen isomerer Decylalkohole nach Anspruch 1 oder 2 dadurch gekennzeichnet, daß die wäßrige Katalysatorlösung ein Phasentransferreagenz enthält.

## Claims

1. A process for preparing mixtures of isomeric decyl alcohols obtained by the hydroformylation of butadiene to form an aldehyde mixture, separation of said aldehyde mixture from the reaction product, condensation of the aldehyde mixture with the formation of an aldol mixture and separation and hydrogenation of said aldol mixture to form a mixture of isomeric decyl alcohols, characterised in that the hydroformylation of the butadiene takes place in the presence of water and water-soluble rhodium complex compounds as the catalyst, said compounds containing sulfonated aryl diphosphines, alkyl diphosphines or alkylaryl diphosphines as ligands.

2. The process for preparing mixtures of isomeric decyl alcohols according to claim 1, characterised in that the hydroformylation of the butadiene takes place in the presence of water and water-soluble rhodium-diphosphine complex compounds as the catalyst at temperatures of 60 to 150°C and at pressures of 0.4 to 30 MPa, the volume ratio of the aqueous to the organic phase is 0.1 to 10:1, the rhodium concentration in the aqueous catalyst solution is 20 to 2000 ppm and 4 to 100 mol of phosphine are used per mole of rhodium.

3. The process for preparing mixtures of isomeric decyl alcohols according to claim 1 or 2, characterised in that the aqueous catalyst solution contains a phase transfer reagent.

## Revendications

1. Procédé de préparation de mélanges d'alcools décyliques isomères obtenus par hydroformylation de butadiène en un mélange d'aldéhydes, par séparation du produit réactionnel du mélange d'aldéhydes obtenu, par condensation du mélange d'aldéhydes avec formation d'un mélange d'aldols et par séparation et hydrogénation du mélange d'aldols en un mélange d'alcools décyliques isomères, caractérisé en ce que l'hydroformylation du butadiène est effectuée en présence d'eau et de composés complexes de rhodium hydrosolubles comme catalyseurs, qui contiennent comme ligands des aryl-, alkyl- ou alkylaryl-diphosphines sulfonées.

2. Procédé de préparation de mélanges d'alcools décyliques isomères selon la revendication 1, caractérisé en ce que l'hydroformylation du butadiène est réalisée en présence d'eau et de composés complexes de rhodium hydrosolubles comme catalyseurs à des températures de 60 à 150°C et sous des pressions de 0,4 à 30 MPa, le rapport en volume de la phase aqueuse à la phase organique étant de 0,1 à 10 : 1 et la concentration de rhodium dans la solution aqueuse de catalyseur étant de 20 à 2 000 ppm et chaque mole de rhodium étant utilisée pour 4 à 100 moles de phosphine.

3. Procédé de préparation de mélanges d'alcools décyliques isomères selon la revendication 1 ou 2, caractérisé en ce que la solution aqueuse de catalyseur contient un agent de transfert de phases.
